(19) Europäisches Patentamt
European Patent Office
Office européen des brevets

(11) Veröffentlichungsnummer: **0 174 271 B1**

# (12) EUROPÄISCHE PATENTSCHRIFT

(45) Veröffentlichungstag der Patentschrift: **16.10.91**

(21) Anmeldenummer: **85810364.1**

(22) Anmeldetag: **05.08.85**

(51) Int. Cl.$^5$: **C07C 22/00**, C07C 33/18, C07C 43/164, C07C 57/30, C07C 59/64, C07C 233/00, C07C 255/00

## (54) Anthracenderivate.

(30) Priorität: **07.09.84 GB 8422665**

(43) Veröffentlichungstag der Anmeldung: **12.03.86 Patentblatt 86/11**

(45) Bekanntmachung des Hinweises auf die Patenterteilung: **16.10.91 Patentblatt 91/42**

(84) Benannte Vertragsstaaten: **CH DE FR GB IT LI NL**

(56) Entgegenhaltungen: **GB-A- 770 222**

(73) Patentinhaber: **CIBA-GEIGY AG**
**Klybeckstrasse 141**
**CH-4002 Basel(CH)**

(72) Erfinder: **Howell, Frederick Harold, Dr.**
**27 High Bank**
**Atherton Lancashire M29 9HZ(GB)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die vorliegende Erfindung betrifft neue Anthracenderivate, insbesondere funktionell alkylierte Verbindungen dieses Typs und deren Verwendung als Sensibilisatoren bei der Photovernetzung von Harzsystemen.

Substituierte Anthracenderivate, deren Substituenten polare Gruppen tragen, sind bekannt. So beschreiben beispielsweise M.S. Newman und S. Otzuka in J. Org. Chem., 23, 797 (1958) 1-Anthrylderivate, die funktionalisierte Alkylsubstituenten tragen. Diese Substituenten können am Kohlenstoffatom, das an den Anthracenring bindet, eine Methylgruppe aufweisen und sie tragen polare Gruppen, wie -COOH, -OH oder -CN. Die Verbindungen werden als Zwischenprodukte zur Synthese von 1,2-Benzanthracenen eingesetzt. P. Arjunan et al. beschreiben in J. Org. Chem., 46, 626 (1981) 2- oder 9-Anthrylalkancarbonsäuren und deren Ester. Die Verbindungen werden als Fluoreszenzindikatoren zur Untersuchung von Membranen eingesetzt.

Die vorliegende Erfindung betrifft Verbindungen der Formel I

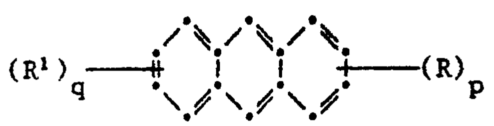 (I),

worin p 1 oder 2 ist, q 0 oder 1 darstellt, mit der Massgabe, dass die Summe p+q 1 oder 2 ist, jeder Substituent R unabhängig voneinander einen Rest der Formel II darstellt

$-A-(Q)_k$ (II),

worin Q einen Rest $-COZR^2$ darstellt, Z -O- oder $-NR^3$- ist, und $R^2$ Wasserstoff, geradkettiges oder verzweigtes $C_1$-$C_{18}$Alkyl, geradkettiges oder verzweigtes $C_3$-$C_{18}$Alkenyl, $C_3$-$C_{12}$Cycloalkyl oder $C_7$-$C_9$ Aralkyl bedeutet, und $R^3$, wenn Z $-NR^3$- ist, Wasserstoff oder geredkettiges oder verzweigtes $C_1$-$C_{18}$Alkyl ist, oder $R^2$ und $R^3$ zusammen mit dem gemeinsamen Stickstoffatom einen fünf- oder sechsgliedrigen heterocyclischen Ring bilden; oder Q ein Rest -OX ist, worin X eine der Bedeutungen von $R^3$ annimmt oder $-COR^4$ ist, und $R^4$ Wasserstoff, geradkettiges oder verzweigtes $C_1$-$C_{18}$Alkyl, geradkettiges oder verzweigtes $C_3$-$C_{18}$Alkenyl oder $C_3$-$C_{12}$Cycloalkyl bedeutet, oder Q ein Rest -CN ist, worin k 1 oder 2 ist, A eine Gruppe der Formel III ist

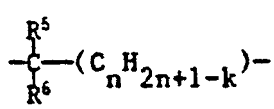 (III),

worin n eine ganze Zahl von 1 bis 17 bedeutet, k die oben definierte Bedeutung besitzt, $R^5$ und $R^6$ gleich oder verschieden sind und geradkettiges oder verzweigtes $C_1$-$C_5$Alkyl bedeuten, und, im Falle von Q = $-COOR^2$, $R^5$ oder $R^6$ gegebenenfalls eine $-COOR^2$ Gruppe tragen können, worin der Rest $R^2$ gleich oder unterschiedlich zum Rest $R^2$ der Gruppe Q ist, und wobei, im Falle der Substitution von $R^5$ oder $R^6$ mit -COOH, die Verbindung der Formel I nicht mehr als drei -COOH Gruppen trägt, oder worin $R^5$ oder $R^6$ so mit dem Rest $C_nH_{2n+1-k}$ verbunden sind, dass ein $C_5$-$C_{12}$Cycloalkylenring entsteht, der den Rest $-(COOR^2)_k$ trägt, worin die Reste $R^2$ und der Index k die oben angegebene Bedeutung besitzten, und der Substituent $R^1$ geradkettiges oder verzweigtes $C_1$-$C_8$Alkyl, $C_7$-$C_9$Aralkyl, das gegebenenfalls ein oder zwei Halogenatome als Substituenten trägt, oder eine $(C_1$-$C_4)$Alkyl$(C_5$-$C_{12})$-cycloalkylgruppe bedeutet; oder worin $R^1$ ein Rest der Formel II, wie oben definiert, ist, wobei im Falle $R^1$ = Rest der Formel II die Substituenten R und $R^1$ gleiche oder unterschiedliche Bedeutung besitzen können; sowie Salze der Verbindungen der Formel I mit organischen oder anorganischen Basen.

Als geradkettige oder verzweigte $C_1$-$C_{18}$Alkylgruppe bedeutet $R^1$ beispielsweise Methyl, Ethyl, n-Propyl, Isopropyl, n-Butyl, Isobutyl, tert.Butyl, tert.Pentyl oder 1,1,3,3-Tetramethylbutyl.

Bedeuten die Reste $R^2$, $R^3$ oder $R^4$ geradkettiges oder verzweigtes $C_1$-$C_{18}$Alkyl, so können sie gleiche oder unterschiedliche Bedeutungen besitzen und stellen beispielsweise Methyl, Ethyl, n-Propyl, Isopropyl, n-Butyl, sek.-Butyl, n-Pentyl, n-Hexyl, n-Heptyl, n-Octyl, 2-Ethylhexyl, n-Nonyl, n-Decyl, n-Dodecyl, n-Hexadecyl oder n-Octadecyl dar.

Die Reste $R^5$ und $R^6$ bedeuten als geradkettiges oder verzweigtes $C_1$-$C_5$Alkyl beispielsweise Methyl, Ethyl, n-Propyl, Isopropyl, n-Butyl, sek.-Butyl oder n-Pentyl. Die Reste können in einer Verbindung gleiche

oder unterschiedliche Bedeutung besitzten.

Bedeuten $R^2$ oder $R^4$ geradkettiges oder verzweigtes $C_3$-$C_{18}$Alkenyl, so handelt es sich dabei beispielsweise um Prop-2-enyl, n-But-2-enyl, 2-Methylprop-2-enyl, n-Pent-2-enyl, n-Hex-2-enyl, n-Hexa-2,4-dienyl, n-Dec-10-enyl, n-Heptadec-8-enyl oder n-Heptadec-8,11-dienyl. Stellen $R^2$ oder $R^4$ eine $C_3$-$C_{12}$Cycloalkylgruppe dar, so handelt es sich dabei beispielsweise um Cyclopropyl, Cyclobutyl, Cyclopentyl, Cyclohexyl, Cyclooctyl oder Cyclododecyl.

Bilden die Reste $R^2$ und $R^3$ zusammen mit dem gemeinsamen Stickstoffatom einen fünf- oder sechsgliedrigen heterocyclischen Ring, so handelt es sich dabei beispielsweise um einen Pyrrolidin-, Piperidin- oder Morpholinring.

Die Gruppe $R^1$ ist als $C_7$-$C_9$Aralkyl, das gegebenenfalls durch ein oder zwei Halogenatome substituiert ist, beispielsweise Benzyl, $\alpha$-Methylbenzyl, p-Chlor-$\alpha$-methylbenzyl, Cumyl, p-Chlorcumyl oder 2,4-Dichlorcumyl.

$R^2$ bedeutet als $C_7$-$C_9$Aralkyl beispielsweise Benzyl, $\alpha$-Methylbenzyl oder Cumyl.

Der Rest $R^1$ ist als ($C_1$-$C_4$)Alkyl($C_5$-$C_{12}$)cycloalkylgruppe beispielsweise 1-Methylcyclopent-1-yl, 1-Methylcyclohex-1-yl, 1-Methylcyclohept-1-yl, 1-Methylcyclooct-1-yl, 1-Ethylcyclohex-1-yl oder 1-n-Butylcyclohex-1-yl.

Zu den Salzen von Verbindungen der Formel I, worin Q eine Säuregruppe, beispielsweise eine -COOH Gruppe, darstellt, zählen beispielsweise Alkali- oder Erdalkalimetallsalze oder Salze mit Aminen.

Beispiele von salzbildenden Aminen, die mit sauren Resten Q umgesetzt werden können, sind aliphatische, cycloaliphatische, aromatische, araliphatische oder heterocyclische Amine.

Bevorzugte Beispiele für aliphatische Amine sind $C_1$-$C_{20}$ aliphatische Amine, wie z.B. Ethylamin, Triethylamin, Decylamin, Dodecylamin oder Octadecylamin.

Bevorzugte Beispiele für cycloaliphatische Amine sind $C_5$-$C_8$cycloaliphatische Amine, wie z.B. Cyclohexylamin.

Bevorzugte Beispiele für aromatische Amine sind Anilin oder Toluidin.

Als araliphatisches Amin lässt sich beispielsweise Benzylamin verwenden.

Zu den bevorzugten heterocyclischen Aminen zählen beispielsweise Morpholin und Pyridin.

Eine bevorzugte Form dieser Erfindung sind Verbindungen der Formel I, worin die Summe $p+q=2$ bedeutet; die Reste R und $R^1$ stellen vorzugsweise jeweils einen Rest der Formel II dar.

Ganz besonders bevorzugt werden Verbindungen der Formel I, worin beide der für R und $R^1$ stehenden Reste der Formel II identisch sind.

Verbindungen dieses Typs enthalten die beiden Reste der Formel II vorzugsweise in 2,6- oder 2,7-Position des Anthracengerüstes gebunden.

Ebenfalls bevorzugt werden Verbindungen der Formel IV

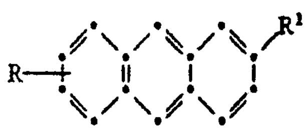

(IV),

worin R einen Rest der Formel II darstellt, wie bereits oben definiert, und $R^1$ ebenfalls ein Rest der Formel II ist oder einen geradkettigen oder verzweigten $C_1$-$C_8$Alkylrest darstellt; sowie deren Salze.

Ferner interessieren Verbindungen der Formel I, worin $p=1$ und $q=0$ ist. Ganz besonders bevorzugt werden Verbindungen der Formel I, worin $k=1$ ist.

Ebenfalls besonders bevorzugt werden Verbindungen der Formel I, worin $k=1$ ist, Q einen Rest $-COOR^2$ darstellt und R die oben definierte Bedeutung besitzt.

Als spezifische Verbindungen der Formel I sind beispielhaft zu erwähnen:

2-(3-Methoxycarbonyl-2-methyl-prop-2-yl)-anthracen
2-(5-Carboxy-2-methyl-pent-2-yl)-anthracen und das Natriumsalz dieser Säure
2-(5-Methoxycarbonyl-2-methyl-pent-2-yl)-anthracen
2-(5-Ethoxycarbonyl-2-methyl-pent-2-yl)-anthracen
2-(5-n-Butoxycarbonyl-2-methyl-pent-2-yl)-anthracen
2-(5-n-Hexyloxycarbonyl-2-methyl-pent-2-yl)-anthracen
2-[5-(2-Ethylhexyloxycarbonyl)-2-methyl-pent-2-yl]anthracen
2-(5-n-Decyloxycarbonyl-2-methyl-pent-2-yl)anthracen
2-(5-n-Octadecyloxycarbonyl-2-methyl-pent-2-yl)-anthracen
2-(7-Methoxycarbonyl-2,6-dimethyl-hept-2-yl)-anthracen

2-(5-N-n-Butylcarbamoyl-2-methyl-pent-2-yl)-anthracen
2-(7-Cyano-2,6-dimethyl-hept-2-yl)-anthracen
2-(6-Hydroxy-2-methyl-hex-2-yl)-anthracen
2-(6-Acetoxy-2-methyl-hex-2-yl)-anthracen
2-(8-Hydroxy-2,6-dimethyl-oct-2-yl)-anthracen
2-(6-Hydroxy-2,6-dimethyl-hept-2-yl)-anthracen
2-(8-Acetoxy-2,6,dimethyl-oct-2-yl)-anthracen
2-(8-Acryloyloxy-2,6-dimethyl-oct-2-yl)-anthracen
2-(8-Methoxy-2,6-dimethyl-hept-2-yl)-anthracen
cis und trans-2-(4-Methoxycarbonyl-1-methyl-cyclohex-1-yl)-anthracen 2-tert-Butyl-6-(3-methoxycarbonyl-2-methyl-prop-2-yl)-anthracen
2-tert-Butyl-7-(3-methoxycarbonyl-2-methyl-prop-2-yl)-anthracen
2-(5-Carboxy-2-methyl-pent-2-yl)-6-methyl-anthracen und das Natriumsalz dieser Säure
2-(5-Carboxy-2-methyl-pent-2-yl)-7-methyl-anthracen und das Natriumsalz dieser Säure
2-(5-Methoxycarbonyl-2-methyl-pent-2-yl)-6-methyl-anthracen
2-(5-Methoxycarbonyl)-2-methyl-pent-2-yl)-7-methyl-anthracen
2-(5-Ethoxycarbonyl-2-methyl-pent-2-yl)-6-methyl-anthracen
2-(5-Ethoxycarbonyl-2-methyl-pent-2-yl)-7-methyl-anthracen
2-(5-n-Butyloxycarbonyl-2-methyl-pent-2-yl)-6-methyl-anthracen
2-(5-n-Butyloxycarbonyl-2-methyl-pent-2-yl)-7-methyl-anthracen
2-(5-n-Hexyloxycarbonyl-2-methyl-pent-2-yl)-6-methyl-anthracen
2-(5-n-Hexyloxycarbonyl-2-methyl-pent-2-yl)-7-methyl-anthracen
2-(5-n-Decyloxycarbonyl-2-methyl-pent-2-yl)-6-methyl-anthracen
2-(5-n-Decyloxycarbonyl-2-methyl-pent-2-yl)-7-methyl-anthracen
2-Ethyl-6-(5-methoxycarbonyl-2-methyl-pent-2-yl)-anthracen
2-Ethyl-7-(5-methoxycarbonyl-2-methyl-pent-2-yl)-anthracen
2-tert-Butyl-6-(5-carboxy-2-methyl-pent-2-yl)-anthracen und das
Natriumsalz dieser Säure
2-tert-Butyl-7-(5-carboxy-2-methyl-pent-2-yl)anthracen und das Natriumsalz dieser Säure
2-tert-Butyl-6-(5-methoxycarbonyl-2-methyl-pent-2-yl)-anthracen
2-tert-Butyl-7-(5-methoxycarbonyl-2-methyl-pent-2-yl)-anthracen
2-tert-Butyl-6-(5-ethoxycarbonyl-2-methyl-pent-2-yl)-anthracen
2-tert-Butyl-7-(5-ethoxycarbonyl-2-methyl-pent-2-yl)-anthracen
2-tert-Butyl-6-(5-n-propyloxycarbonyl-2-methyl-pent-2-yl)-anthracen
2-tert-Butyl-7-(5-n-propyloxycarbonyl-2-methyl-pent-2-yl)-anthracen
2-tert-Butyl-6-(5-isopropyloxycarbonyl-2-methyl-pent-2-yl)-anthracen
2-tert-Butyl-7-(5-isopropyloxycarbonyl-2-methyl-pent-2-yl)-anthracen
2-tert-Butyl-6-(5-n-butyloxycarbonyl-2-methyl-pent-2-yl)-anthracen
2-tert-Butyl-7-(5-n-butyloxycarbonyl-2-methyl-pent-2-yl)-anthracen
2-tert-Butyl-6-(5-n-hexyloxycarbonyl-2-methyl-pent-2-yl)-anthracen
2-tert-Butyl-7-(5-n-hexyloxycarbonyl-2-methyl-pent-2-yl)-anthracen
2-tert-Butyl-6-[5-(2-ethylhexyloxycarbonyl)-2-methyl-pent-2-yl]-anthracen
2-tert-Butyl-7-[5-(2-ethylhexyloxycarbonyl)-2-methyl-pent-2-yl]-anthracen
2-tert-Butyl-6-(n-decyloxycarbonyl-2-methyl-pent-2-yl)-anthracen
2-tert-Butyl-7-(n-decyloxycarbonyl-2-methyl-pent-2-yl)-anthracen
2-tert-Butyl-6-(cylcohexyloxycarbonyl-2-methyl-pent-2-yl)-anthracen
2-tert-Butyl-6-(5-carbamoyl-2-methyl-pent-2-yl)-anthracen
2-tert-Butyl-6-(5-N-methylcarbamoyl-2-methyl-pent-2-yl)-anthracen
2-(1,1,3,3-Tetramethylbutyl)-6-(5-methoxycarbonyl-2-methyl-pent-2-yl)-anthracen
2-(1,1,3,3-Tetramethylbutyl)-7-(5-methoxycarbonyl-2-methyl-pent-2-yl)-anthracen
2-Cumyl-6-(5-methoxycarbonyl-2-methyl-pent-2-yl)-anthracen
2-Cumyl-7-(5-methoxycarbonyl-2-methyl-pent-2-yl)-anthracen
2-(1-Methyl-cyclohex-1-yl)-6-(5-methoxycarbonyl-2-methyl-pent-2-yl)-anthracen
2-(1-Methyl-cyclohex-1-yl)-7-(5-methoxycarbonyl-2-methyl-pent-2-yl)-anthracen
2-tert-Butyl-6-(7-methoxycarbonyl-2,6-dimethyl-hept-2-yl)-anthracen
2-tert-Butyl-7-(7-methoxycarbonyl-2,6-dimethyl-hept-2-yl)-anthracen
2-tert-Butyl-6-(7-cyano-2,6-dimethyl-hept-2-yl)-anthracen
2-tert-Butyl-7-(7-cyano-2,6-dimethyl-hept-2-yl)-anthracen

2-tert-Butyl-6-(8-hydroxy-2,6-dimethyl-oct-2-yl)-anthracen
2-tert-Butyl-7-(8-hydroxy-2,6-dimethyl-oct-2-yl)-anthracen
2-tert-Butyl-6-(8-acetoxy-2,6-dimethyl-oct-2-yl)-anthracen
2-tert-Butyl-7-(8-acetoxy-2,6-dimethyl-oct-2-yl)-anthracen cis und trans 2-tert-Butyl-6-(4-methoxycarbonyl-1-methyl-cyclohex-1-yl)-anthracen
cis und trans 2-tert-Butyl-7-(4-methoxycarbonyl-1-methyl-cyclohex-1-yl)-anthracen
2,6-Bis-(3-methoxycarbonyl-2-methyl-prop-2-yl)-anthracen
2,7-Bis-(3-methoxycarbonyl-2-methyl-prop-2-yl)-anthracen
2,6-Bis-(5-carboxy-2-methyl-pent-2-yl)-anthracen und das Natriumsalz dieser Säure
2,7-Bis-(5-carboxy-2-methyl-pent-2-yl)-anthracen und das Natriumsalz dieser Säure
2,6-Bis-(5-methoxycarbonyl-2-methyl-pent-2-yl)-anthracen
2,7-Bis-(5-methoxycarbonyl-2-methyl-pent-2-yl)-anthracen
2,6-Bis-(5-ethoxycarbonyl-2-methyl-pent-2-yl)-anthracen
2,7-Bis-(5-ethoxycarbonyl-2-methyl-pent-2-yl)-anthracen
2,6-Bis-(5-n-propyloxycarbonyl-2-methyl-pent-2-yl)-anthracen
2,7-Bis-(5-n-propyloxycarbonyl-2-methyl-pent-2-yl)-anthracen
2,6-Bis-(5-isopropyloxycarbonyl-2-methyl-pent-2-yl)-anthracen
2,7-Bis-(5-isopropyloxycarbonyl-2-methyl-pent-2-yl)-anthracen
2,6-Bis-(5-n-butyloxycarbonyl-2-methyl-pent-2-yl)-anthracen
2,7-Bis-(5-n-butyloxycarbonyl-2-methyl-pent-2-yl)-anthracen
2,6-Bis-(5-n-hexyloxycarbonyl-2-methyl-pent-2-yl)-anthracen
2,7-Bis-(5-n-hexyloxycarbonyl-2-methyl-pent-2-yl)-anthracen
2,6-Bis-[5-(2-ethylhexyloxycarbonyl)-2-methyl-pent-2-yl]anthracen
2,7-Bis-[5-(2-ethylhexyloxycarbonyl)-2-methyl-pent-2-yl]anthracen
2,6-Bis-(5-n-decyloxycarbonyl-2-methyl-pent-2-yl)-anthracen
2,7-Bis-(5-n-decyloxycarbonyl-2-methyl-pent-2-yl)-anthracen
2,6-Bis-(7-methoxycarbonyl-2,6-dimethyl-hept-2-yl)-anthracen
2,7-Bis-(7-methoxycarbonyl-2,6-dimethyl-hept-2-yl)-anthracen
2,6-Bis-(5-N,N-dimethylcarbamoyl-2-methyl-pent-2-yl)-anthracen
2,6-Bis-(5-N-n-butylcarbamoyl-2-methyl-pent-2-yl)-anthracen
2,7-Bis-(5-N-n-butylcarbamoyl-2-methyl-pent-2-yl)-anthracen
2,7-Bis-(5-N-allylcarbamoyl-2-methyl-pent-2-yl)-anthracen
2,7-Bis-(5-N-cyclohexylcarbamoyl-2-methyl-pent-2-yl)-anthracen
2,7-Bis-(5-N-benzylcarbamoyl-2-methyl-pent-2-yl)-anthracen
2,7-Bis-(5-morpholinocarbonyl-2-methyl-pent-2-yl)-anthracen
2,6-Bis-(7-cyano-2,6-dimethyl-hept-2-yl)-anthracen
2,7-Bis-(7-cyano-2,6-dimethyl-hept-2-yl)-anthracen
2,6-Bis-(8-methoxy-2,6-dimethyl-hept-2-yl)-anthracen
2,7-Bis-(8-methoxy-2,6-dimethyl-hept-2-yl)-anthracen
2,6-Bis-(8-hydroxy-2,6-dimethyl-hept-2-yl)-anthracen
2,7-Bis-(8-hydroxy-2,6-dimethyl-hept-2-yl)-anthracen
2,6-Bis-(8-acetoxy-2,6-dimethyl-hept-2-yl)-anthracen
2,7-Bis-(8-acetoxy-2,6-dimethyl-hept-2-yl)-anthracen
2,6-Bis-(8-acryloyloxy-2,6-dimethyl-hept-2-yl)-anthracen
2,6-Bis-(8-cyclohexanoyloxy-2,6-dimethyl-hept-2-yl)-anthracen
2,6-Bis-(4-methoxycarbonyl-1-methyl-cyclohex-1-yl)-anthracen
2,7-Bis-(4-methoxycarbonyl-1-methyl-cyclohex-1-yl)-anthracen.

Die Verbindungen der Formel I können nach an sich bekannten Verfahren hergestellt werden.

So kann man sie beispielsweise durch funktionelle Alkylierung von Anthracen(derivaten) der Formel I, worin p 0 ist, wie z.B. Anthracen, 2-Methylanthracen, 2-tert.Butylanthracen, 2-(3-Methoxycarbonyl-2-methyl-prop-2-yl)-anthracen oder 2-(5-Methoxycarbonyl-2-methylpent-2-yl)-anthracen erhalten.

Ein weiterer möglicher Syntheseweg zu den Verbindungen der Formel I verläuft über ein Zweistufenverfahren. Dazu wird zunächst ein Anthracen(derivat) der Formel I, worin p 0 ist, wie beispielsweise Anthracen, 2-Methylanthracen, 2-tert.Butylanthracen, 2-(5-Methoxycarbonyl-2-methyl-pent-2-yl)-anthracen oder 2-(3-Carboxypropanoyl)-anthracen, mit einem geeigneten Acylierungsmittel, wie beispielsweise Bernsteinsäureanhydrid, Glutarsäureanhydrid, Methyl-3-chlorformylpropanoat, Ethyl-5-chlorformylpentanoat oder Methyl-9-chlorformylnonanoat, umgesetzt. In einer weiteren Verfahrensstufe wird dann der Acylcarbonylrest mittels an sich bekannter Verfahren in einen Kohlenwasserstoffrest umgewandelt. Ein Beispiel für solche

Umwandlungen in einen Kohlenwasserstoffrest ist die Ueberführung einer Carbonylgruppe in eine $=C(CH_3)_2$ Gruppe mittels geminaler Alkylierung. Die geminale Alkylierung der Carbonylgruppe kann beispielsweise nach der von Weidmann und Seebach beschriebenen Methode (Angew. Chem. Int. Ed., Engl. 22, 31-45 (1983)) durchgeführt werden.

Enthalten die Verbindungen der Formel I zwei Reste R oder einen Rest R und einen weiteren Rest $R^1$, so kann die funktionelle Alkylierung und/oder die Acylierungen und Ueberführung in den Kohlenwasserstoffrest schrittweise durchgeführt werden.

Sind die beiden Reste R oder die zwei Reste R und $R^1$ identisch, so ist es angemessen, diese gleichzeitig in einem Schritt einzuführen.

Monosubstituierte Anthracene, die keine funktionelle Gruppe tragen, und die als Zwischenprodukte einsetzbar sind, sind an sich bekannt und können nach herkömmlichen Verfahren hergestellt werden. Solche Verfahren sind beispielsweise im US-Patent Nr. 4,255,343, in 'Friedel Crafts and Related Reactions, Bd. III'; Herausgeber: G. Olah oder In Houben Weyl, Band VII/Teil 2a, S. 76 ff. beschrieben.

Die funktionelle Alkylierung von gegebenenfalls substituiertem Anthracen zur Herstellung der Verbindungen der Formel I hat den Vorteil, ein Einstufenverfahren zu sein. Die Reaktion wird üblicherweise im Temperaturbereich von 20-170 °C durchgeführt, vorzugsweise zwischen 20 und 120 °C.

Die funktionelle Acylierung wird ebenfalls üblicherweise bei erhöhten Temperaturen durchgeführt; in der Regel im oben definierten Temperaturbereich.

Die Umsetzung wird in Anwesenheit eines Katalysators, beispielsweise einer Broenstedt Säure, einer aktivierten Erde oder eines Metallsalzes oder einer Kombination von Metallsalz mit Broenstedt Säure durchgeführt. Als Broenstedt Säuren verwendet man organische oder anorganische Säuren oder partielle Salze dieser Säuren. Beispiele dafür sind anorganische Mineralsäuren, wie z.B. Salzsäure, Schwefelsäure, Perchlorsäure oder Orthophosphorsäure; sowie alkyl-, halogen-alkyl-, aryl- oder alkarylsubstituierte anorganische Säuren, wie z.B. Methan-, Difluormethan-, Trifluormethan- oder Ethansulfonsäure, Benzolsulfonsäure, p-Toluolsulfonsäure oder Methanphosphonsäure; oder man setzt organische Säuren ein, wie z.B. Dichloressigsäure, Trichloressigsäure oder Trifluoressigsäure.

Als aktivierte Erden kann man solche Katalysatoren verwenden, die kommerziell verfügbar sind, beispielsweise unter den Handelsbezeichnungen Fulmont® 237 oder Fulcat® 22; als Metallsalzkatalysatoren eignen sich beispielsweise Aluminiumchlorid, Zinkchlorid oder Zinnchlorid.

Je nachdem, ob ein oder zwei funktionelle Alkyl- oder Acylgruppen eingeführt werden sollen, werden bis zu einem Mol oder wenigstens zwei Mole des funktionellen Alkylierungsmittels oder des funktionellen Acylierungsmittels pro Mol Anthracen eingesetzt.

Soll eine Alkyl-, Acyl- oder funktionelle Alkylgruppe in ein substituiertes Anthracenderivat eingeführt werden, so werden bis zu einem Mol Alkylierungsmittel-, Acylierungsmittel oder funktionelles Alkylierungsmittel pro Mol Anthracenderivat eingesetzt. Die Umsetzung kann in An- oder Abwesenheit eines Lösungsmittels durchgeführt werden. Beispiele für geeignete Lösungsmittel sind Benzol, Chlorbenzol, Toluol, Xylol, Nitromethan oder Essigsäure.

Unter dem Begriff 'funktionelles Alkylierungsmittel' versteht man Verbindungen mit einem reaktiven Zentrum, beispielsweise einer olefinischen Gruppe oder einer Hydroxygruppe.

Beispiele für geeignete olefinische funktionelle Alkylierungsmittel sind:
5-Methylhex-5-ensäure
Methyl-5-methyl-hex-4-enoat
Methyl-5-methylhex-5-enoat
Ethyl-5-methylhex-5-enoat
n-Propyl-5-methylhex-5-enoat
iso-Propyl-5-methylhex-5-enoat
n-Butyl-5-methylhex-5-enoat
iso-Butyl-5-methylhex-5-enoat
sek-Butyl-5-methylhex-5-enoat
n-Pentyl-5-methylhex-5-enoat
iso-Pentyl-5-methylhex-5-enoat
sek-Pentyl-5-methylhex-5-enoat
n-Hexyl-5-methylhex-5-enoat
Cyclohexyl-5-methylhex-5-enoat
2-Ethylhexyl-5-methylhex-5-enoat
n-Octyl-5-methylhex-5-enoat
Methyl-5,7,7-trimethyl-oct-4-enoat
1,7-Di-methoxycarbonyl-4-methyl-hept-3-en

4-Carbomethoxy-1-methylcyclohex-1-en
4-Acetyl-1-methylcyclo-hex-1-en
Methyl-citronellat
Citronellol
Citronellyl-acetat
Citronellyl-methyl-ether
Citronellyl-n-butyl-ether
Citronellyl-nitril
Ethyl-2-ethoxycarbonyl-5-methyl-hex-4-enoat.

Beispiele für geeignete Alkylierungsmittel sind Isobutylen, Diisobutylen, tert.Butanol, tert.Butylacetat, 1,1,3,3-Tetramethylbutan-1-ol, 1-Methylcyclohex-1-en, α-Methylstyrol, Cumylalkohol oder Cumylacetat.

Ein beliebiges funktionelles Derivat einer Verbindung der Formel I kann in ein anderes funktionelles Derivat übergeführt werden. So lässt sich beispielsweise Q als Carbonsäurerest -COOH mit einem Alkohol $R^2OH$ in den entsprechenden Carbonsäureester, worin Q $-COOR^2$ bedeutet, überführen; oder Q als Esterrest $-COOR^2$ lässt sich durch Umesterung in einen anderen Ester mit einer anderen Gruppe $R^2$ überführen; oder die Estergruppe $-COOR^2$ lässt sich in ein Amid $-CONR^2R^3$ umwandeln, indem man besagten Ester mit dem Amin $NHR^2R^3$ behandelt; dabei besitzen $R^2$ und $R^3$ die schon weiter oben definierten Bedeutungen; oder eine Estergruppe $-COOR^2$ lässt sich durch Reduktion mit einem Grignard-Reagens in einen Alkohol überführen. Stellt Q einen Alkoholrest dar, so kann dieser gegebenenfalls mit einem Acylierungsmittel verestert und in eine Gruppierung $-OCOR^4$ übergeführt werden, worin $R^4$ die bereits oben angegebene Bedeutung besitzt.

Die Verbindungen der Formel I lassen sich als Sensibilisatoren für photovernetzbare Polymersysteme verwenden. Solche Systeme werden bevorzugt zur Herstellung von Schutzüberzügen oder bei der Herstellung lichtinduzierter Abbildungen eingesetzt.

Die folgenden Beispiele beschreiben die vorliegende Erfindung näher ohne sie zu begrenzen. Die Mengenangaben beziehen sich jeweils auf Gewichtsteile.

Beispiel 1: Herstellung von 2,6- und 2,7-Bis-(5-methoxycarbonyl-2-methylpent-2-yl)-anthracen

Zu einer Lösung von 15,0 Teilen Aluminiumchlorid in 50 Teilen Nitromethan gibt man bei Raumtemperatur unter Rühren 8,9 Teile Anthracen und tropft anschliessend 14,2 Teile Methyl-5-methylhex-5-enoat über einen Zeitraum von 15 Minuten zu. Nach Vervollständigung der Addition wird weitere 24 Stunden bei Raumtemperatur gerührt und die Reaktionsmischung anschliessend in 150 Teile 2N-Salzsäure gegossen.

Die organische Phase wird mit Ether extrahiert, der Etherextrakt mit Wasser gewaschen und der Ether verdampft. Das zurückbleibende Oel wird dann 3 Stunden lang mit 200 Teilen Methanol, das zuvor mit Chlorwasserstoffgas gesättigt wurde, gekocht. Das Methanol wird nun mit einem Rotationsverdampfer abgezogen und das zurückbleibende Oel wird mit Ether verdünnt. Die Etherlösung wird mit Natriumbicarbonatlösung gewaschen, anschliessend mit Wasser ausgeschüttelt und dann konzentriert. Destillation des Rückstandes ergibt eine Fraktion mit des Siedebereich bei 0,7 mbar von 250-300° C. Diese Fraktion enthält 94 % eines Isomerengemisches aus 2,6- und 2,7-Bis-(5-methoxycarbonyl-2-methylpent-2-yl)-anthracen. Fraktionierte Kristallisation aus Methanol ergibt als erste Fraktion das kristalline 2,6-Isomere mit einem F.P. von 111-113° C mit den folgenden Elementaranalysedaten:

| | C | H |
|---|---|---|
| Berechnet für $C_{30}H_{38}O_4$ | 77,89 | 8,28 |
| Gefunden | 77,82 | 8,65 |

Die Mutterlaugen des obigen Kristallisationsprozesses werden konzentriert und ergeben dann als zweite Fraktion Kristalle des 2,7-Isomers mit dem F.P. von 94-96° C und den folgenden Elementaranalysedaten:

| | C | H |
|---|---|---|
| Berechnet für $C_{30}H_{38}O_4$ | 77,89 | 8,28 |
| Gefunden | 77,94 | 8,38 |

Beispiel 2: Herstellung von 2-(5-Methoxycarbonyl-2-methylpent-2-yl)-anthracen

8,9 Teile Anthracen, 14,2 Teile Methyl-5-methylhex-5-enoat und 26,1 Teile Zinnchlorid werden zusammen mit 50 Teilen p-Xylol 24 Stunden lang auf dem Dampfbad erhitzt. Die Reaktionsmischung wird anschliessend in 250 Teile 2N-Salzsäure gegossen und die organische Phase mit Ether extrahiert. Die Etherlösung wird abwechselnd mit Wasser, Natriumbicarbonatlösung und wieder mit Wasser gewaschen und anschliessend wird der Ether verdampft. Der Rückstand wird in 100 Teilen Methanol aufgenommen und 3,7 Teile Anthracen werden durch Filtration von dieser Lösung abgetrennt. Die Methanollösung wird unter verminderten Druck eingedampft und der verbleibende Rückstand wird destilliert. Man erhält eine Fraktion mit dem Siedepunkt bei 0,7 mbar von 220 °C.

Das Destillat wird aus Petrolether mit dem Siedebereich 40-60 °C umkristallisiert und man erhält das 2-(5-Methoxycarbonyl-2-methylpent-2-yl)-anthracen mit dem F. P. von 82-84 °C und den folgenden Elementaranalysedaten:

| | C | H |
|---|---|---|
| Berechnet für $C_{22}H_{24}O_2$ | 82,46 | 7,55 |
| Gefunden | 82,59 | 7,79 |

Beispiel 3: Herstellung von 2-, 2,6- und 2,7-(Bis)-(5-carboxy-2-methyl-pent-2-yl)-anthracen bzw. der entsprechenden Methylester

21,6 Teile wasserfreien Zinnchlorids werden in 100 Teilen p-Xylol gelöst. Zu dieser Lösung gibt man 1,5 Teile Trifluormethansulfonsäure, 14,2 Teile Methyl-5-methylhex-5-enoat und 17,8 Teile Anthracen. Diese Mischung wird anschliessend 8 Stunden lang auf dem Dampfbad erhitzt und dann in 500 Teile Wasser gegossen. Die organische Phase wird mit Ether aufgenommen, die Etherlösung mit Wasser gewaschen und eingedampft. Der Rückstand wird mit 300 ml Methanol versetzt, unter Rückfluss gekocht und dann filtriert, wobei 6,7 Teile unlösliches Anthracen abgetrennt werden.

Zum Filtrat gibt man 1,0 Teile p-Toluolsulfonsäure und kocht anschliessend 4 Stunden lang unter Rückfluss, um die bei der Aufarbeitung entstandene freie Säure zu verestern. Nach Entfernen des Methanols unter vermindertem Druck wird der Rückstand in Ether aufgenommen, die Etherlösung mit Natriumbicarbonatlösung und anschliessend mit Wasser gewaschen und der Ether verdampft. Destillation des Rückstandes ergibt eine erste Fraktion vom Siedepunkt (0,5 mbar) 210-240 °C. Diese enthält 2-(5-Methoxycarbonyl-2-methyl-pent-2-yl)-anthracen vom F.P. 82-84 °C. Dieses Produkt ist identisch mit der in Beispiel 2 hergestellten Verbindung.

Eine zweite Fraktion mit dem Siedebereich (0,5 mbar) 240-290 °C enthält eine Mischung von 2,6- und 2,7-Bis-(5-methoxycarbonyl-2-methyl-pent-2-yl)-anthracen.

Diese Mischung wird durch fraktionierte Kristallisation aufgearbeitet. Als Lösungsmittel verwendet man Methanol. Man erhält die 2,6- bzw. 2,7-Isomeren mit dem F.P. 111-113 °C bzw. 94-96 °C, die identisch mit den in Beispiel 1 hergestellten Produkten sind.

Die drei Ester dieses Beispiels können durch alkalische Hydrolyse in ihre jeweiligen Carbonsäuren übergeführt werden. Diese Säuren besitzen folgende Schmelzpunkte bzw. Analysedaten:

| | F.P. (°C) | | Elementaranalyse C | H |
|---|---|---|---|---|
| 2-(5-Carboxy-2-methyl-pent-2-yl)anthracen | 149-151 | gefunden | 81,95 | 7,29 |
| | | berechnet | 82,32 | 7,24 |
| 2,6-Bis-(5-carboxy-2-methyl-pent-2-yl)-anthracen | 263-265 | gefunden | 77,52 | 7,66 |
| | | berechnet | 77,39 | 7,89 |
| 2,7-Bis-(5-carboxy-2-methyl-pent-2-yl)-anthracen | 159-161 | gefunden | 77,40 | 8,12 |
| | | berechnet | 77,39 | 7,89 |

Beispiel 4: Herstellung von 2-, 2,6- und 2,7-(Bis)-(7-carboxy-2,6-dimethyl-hept-2-yl)-anthracen

8,9 Teile Anthracen, 18,4 Teile Methylcitronellat, 13,0 Teile Zinnchlorid und 1,5 Teile Trifluormethansulfonsäure werden in 50 Teilen p-Xylol auf dem Dampfbad 24 Stunden lang erhitzt. Man arbeitet wie in Beispiel 1 beschrieben auf und erhält nach Destillation eine Fraktion mit dem K.P. (0,3 mbar) 220-280 °C. Diese Fraktion enthält zu 84 % 2-(7-Methoxycarbonyl-2,6-dimethyl-hept-2-yl)-anthracen. Der Ester wird als weisser kristalliner Feststoff vom F.P. 41-43 °C erhalten. Die Reinigung erfolgt durch Umkristallisieren aus Petrolether vom Siedebereich 40-60 °C und anschliessende Säulenchromatographie an Kieselgel mit Petrolether vom Siedebereich 40-60 °C.

| Elementaranalyse: | C | H |
|---|---|---|
| gefunden | 82,75 | 8,17 |
| berechnet für $C_{25}H_{30}O_2$ | 82,83 | 8,34 |

Dieser Ester lässt sich durch alkalische Hydrolyse in die entsprechende Carbonsäure überführen. Nach Umkristallisieren aus Essigsäure, die etwas Wasser enthält, fällt 2-(7-Carboxy-2,6-dimethyl-hept-2-yl)-anthracen als Feststoff vom F.P. 127-129 °C an.

| Elementaranalyse: | C | H |
|---|---|---|
| gefunden | 82,70 | 7,76 |
| berechnet für $C_{24}H_{28}O_2$ | 82,72 | 8,10 |

Führt man die oben beschriebene Destillation fort, so erhält man eine weitere Fraktion mit dem Siedebereich (0,6 mbar) 290-310 °C. Diese Fraktion enthält eine Mischung von 2,6- und 2,7-Bis-(7-methoxycarbonyl-2,6-dimethyl-hept-2-yl)anthracen.

| Elementaranalyse: | C | H |
|---|---|---|
| gefunden | 79,38 | 9,19 |
| berechnet für $C_{36}H_{50}O_4$ | 79,08 | 9,22 |

Beispiel 5: Herstellung von cis- und trans-2-(4-Methoxycarbonyl-1-methyl-cyclohex-1-yl)-anthracen bzw. des entsprechenden 2,6- und 2,7-disubstituierten Monomeren

Das Verfahren von Beispiel 3 wird wiederholt, indem man 30,8 Teile 4-Methoxycarbonyl-1-methyl-cyclohex-1-en anstelle des Methyl-5-methyl-hex-5-enoats einsetzt, und die Reaktionsmischung 24 Stunden lang reagieren lässt.

Durch Destillation erhält man eine Fraktion vom Siedebereich 200-260 °C (0,5 mbar), die zu 91 % aus einem Isomerengemisch aus cis- und trans-2-(4-Methoxycarbonyl-1-methyl-cylcohex-1-yl)-anthracen besteht. Das Destillat wird mit einer Mischung aus Ether und Petrolether (Siedebereich 40-60 °C) behandelt und man erhält einen Feststoff bestehend aus fast gleichen Anteilen des cis- und des trans-Isomeren. Dieses Isomerengemisch wird dann durch fraktionierte Kristallisation aus Methanol getrennt. Das cis-Isomere besitzt einen F.P. von 178-181 °C und das trans-Isomere besitzt einen F.P. von 139-142 °C.

Folgende Elementaranalysedaten werden ermittelt:

| | C | H |
|---|---|---|
| berechnet für $C_{23}H_{24}O_2$ | 83,10 | 7,28 |
| gefunden für das trans-Isomer | 83,28 | 7,42 |
| gefunden für das cis-Isomer | 83,13 | 7,43 |

Die Destillation wird fortgesetzt und man erhält eine Fraktion vom Siedebereich (0,5 mbar) 260-350 °C, die mit Ether aufgenommen wird, konzentriert wird und mit Petrolether vom Siedebereich 40-60 °C verdünnt wird.

Man erhält eine Mischung des 2,6-und 2,7-Bis-(4-methoxycarbonyl-1-methyl-cyclohex-1-yl)-anthracens vom F.P. 176-214 °C.

| Elementaranalyse: | C | H |
|---|---|---|
| berechnet für $C_{32}H_{38}O_4$ | 78,98 | 7,87 |
| gefunden | 78,83 | 7,91 |

Beispiel 6: Herstellung von 2-tert.Butyl-6- und -7-(5-methoxy-carbonyl-2-methyl-pent-2-yl)anthracen

Durch eine Lösung aus 3,2 Teilen 2-(5-Methoxycarbonyl-2-methyl-pent-2-yl)-anthracen, 2,6 Teilen Zinnchlorid und 0,15 Teilen Trifluormethansulfonsäure in 25 Teilen p-Xylol wird bei 100 °C 1 Stunde lang Isobutylengas geleitet. Dabei wird gerührt. Die Reaktionsmischung wird dann in 250 Teile Wasser gegossen und die organische Phase mit Ether isoliert.

Durch Destillation isoliert man eine Fraktion mit dem Siedebereich (0,5 mbar) 190-220 °C. Nach Verdünnen mit Petrolether (Siedebereich 40-60 °C) und Lagerung bei -20 °C isoliert man eine Mischung aus 2-tert.-Butyl-6- und -7-(5-methoxycarbonyl-2-methyl-pent-2-yl)-anthracen vom F.P. 81-89 °C.

| Elementaranalyse: | C | H |
|---|---|---|
| berechnet für $C_{26}H_{32}O_2$ | 82,94 | 8,57 |
| gefunden | 83,27 | 8,60 |

Beispiel 7: Herstellung von 2-(6-Hydroxy-2,6-dimethyl-hept-2-yl)-anthracen

13,5 Teile 2-(5-Methoxycarbonyl-2-methyl-pent-2-yl)-anthracen in 100 Teilen Ether werden zu einer Lösung aus Methylmagnesiumiodid gegeben, die durch Umsetzung von 3,0 Teilen Magnesium und 18,0 Teilen Methyliodid in 150 Teilen Ether erhalten wurde. Nach beendeter Addition wird die Reaktionsmischung 3 Stunden lang gerührt und anschliessend mit verdünnter Salzsäure behandelt. Die Etherphase wird abgetrennt, mit Wasser gewaschen und der Ether abgezogen. Nach Umkristallisieren aus Petrolether (Siedebereich 60-80 ° C) verbleibt 2-(6-Hydroxy-2,6-dimethyl-hept-2-yl)-anthracen vom F.P. 117-118 ° C

| Elementaranalyse: | C | H |
|---|---|---|
| berechnet für $C_{23}H_{28}O$ | 86,20 | 8,81 |
| gefunden | 86,48 | 8,94 |

Beispiel 8: Herstellung von 2-(6-Hydroxy-2-methyl-hex-2-yl)-anthracen

1,1 Teile 2-(5-Methoxycarbonyl-2-pent-2-yl)-anthracen und 1,0 Teile Lithiumaluminiumhydrid werden in 50 Teilen von über Natrium getrocknetem Ether über Nacht unter Rühren stehen gelassen. Anschliessend wird mit verdünnter Salzsäure behandelt. Die Etherphase wird abgetrennt, mit Wasser gewaschen, eingedampft und der Rückstand aus Petrolether (Siedebereich 40-60 ° C) enthaltend etwas Aceton umkristallisiert. Man erhält 2-(6-Hydroxy-2-methyl-hex-2-yl)anthracen mit dem F.P. 123-125 ° C

| Elementaranalyse: | C | H |
|---|---|---|
| berechnet für $C_{21}H_{24}O$ | 86,26 | 8,27 |
| gefunden | 86,31 | 8,25 |

Der Alkohol kann durch Behandlung mit Essigsäureanhydrid in das 2-(6-Acetoxy-2-methyl-hex-2-yl)-anthracen übergeführt werden. Die Verbindung besitzt nach dem Umkristallisieren aus Petrolether (Siedebereich 40-60 ° C) bei 0 ° C eine F.P. von 58-59 ° C.

| Elementaranalyse: | C | H |
|---|---|---|
| berechnet für $C_{23}H_{26}O_2$ | 82,60 | 7,84 |
| gefunden | 82,01 | 7,84 |

Beispiel 9: Herstellung von 2-(5-N-n-Butylcarbamoyl-2-yl)-anthracen

1,0 Teile 2-(5-Methoxycarbonyl-2-methyl-pent-2-yl)-anthracen und 3,0 Teile n-Butylamin werden in eine Glasphiole eingeschmolzen und 24 Stunden lang auf 120 ° C erhitzt. Das überschüssige Amin wird verdampft und der Rückstand aus Ether bei 0 ° C umkristallisiert. Man erhält 2-(5-N-n-Butylcarbamoyl-2-methyl-pent-2-yl)-anthracen vom F.P. 111-113 ° C.

| Elementaranalyse: | C | H | N |
|---|---|---|---|
| berechnet für $C_{25}H_{31}NO$ | 83,06 | 8,64 | 3,87 |
| gefunden | 83,04 | 8,66 | 3,69 |

Beispiel 10: Herstellung von 2,6-Bis-(6-hydroxy-2-methyl-hex-2-yl)-anthracen

1,0 Teile 2,6-Bis-(5-methoxycarbonyl-2-methyl-pent-2-yl)-anthracen und 1,0 Teile Lithiumaluminiumhydrid werden in 25 Teilen trockenem Ether reagieren gelassen und aufgearbeitet, wie in beispiel 8 beschrieben. Als Reduktionsprodukt erhält man 2,6-Bis-(6-hydroxy-2-methyl-hex-2-yl)-anthracen. Nach Umkristallisation aus Aceton besitzt dieses Produkt einen F.P. von 174-176 ° C.

| Elementaranalyse: | C | H |
|---|---|---|
| berechnet für $C_{28}H_{38}O_2$ | 82,71 | 9,42 |
| gefunden | 82,62 | 9,28 |

Beispiel 11: Herstellung von 2,6-Bis-(5-N-n-Butylcarbamoyl-2-methyl-pent-2-yl)anthracen

0,25 Teile 2,6-Bis-(5-methoxycarbonyl-2-methyl-pentyl-2-yl)-anthracen und 2,0 Teile n-Butylamin werden miteinander umgesetzt und aufgearbeitet, wie in Beispiel 9 beschrieben. Nach dem Umkristallisieren aus Ether besitzt das 2,6-Bis-(5-N-n-Butylcarbamoyl-2-methyl-pent-2-yl)-anthracen einen F.P. von 153-155 °C.

| Elementaranalyse: | C | H | N |
|---|---|---|---|
| berechnet für $C_{36}H_{52}N_2O_2$ | 79,36 | 9,62 | 5,14 |
| gefunden | 79,05 | 9,72 | 4,91 |

Anwendungsbeispiele

Beispiel I: Eine Lösung bestehend aus 10 g eines technischen Epoxidkresolnovolak (Epoxidgehalt 4,5 Aeq/kg), 0,25 g (7 x $10^{-4}$ Mol) ($\eta^6$-Toluol)-($\eta^5$-cyclopentadienyl)-eisen(II)-hexafluorophosphat [hergestellt gemäss Bull. Soc. Chim. France, 2572 (1975)] 1,3 x $10^{-3}$ Mol 2,6-Bis-(5-methoxycarbonyl-2-methyl-pent-2-yl)-anthracen und 4 g 1-Acetoxy-2-ethoxyethan wird mit einem 25 µ Drahtrakel auf eine kupferkaschierte Epoxidplatte aufgebracht. Der vorerst nasse Film wird bei 80 °C getrocknet (Trockenfilmdicke ca. 15 µ). Die Belichtung der so hergestellten Photopolymerplatte erfolgt mit einer 5000 Watt Quecksilberhochdrucklampe, Abstand zum Probentisch 50 cm, wobei als Vorlage ein 21 step Stouffer sensitivity guide verwendet wird (s. W.S. DeForest, Photoresist, McGraw-Hill Book Compl, N.Y., S. 109 ff). Die Belichtungszeit beträgt 30 sec., danach wird die belichtete Platte während 2 Minuten bei 110 °C gehärtet. Die Entwicklung erfolgt in 1-Acetoxy-2-ethoxyethan, wobei die nicht belichteten Anteile gelöst werden. Es resultiert ein Reliefbild, mit der letzten abgebildeten Stufe No. 5.

Beispiel II: Das Verfahren von Beispiel I wird wiederholt. Nur verwendet man anstelle des 2,6-Bis-(5-methoxycarbonyl-2-methyl-pent-2-yl)-anthracens das entsprechende 2,7-Isomere. Die Sensibilisatormenge beträgt hier ebenfalls 1,3 x $10^{-3}$ Mol.

Es wird je 10, 20 und 30 Sekunden lang belichtet. Es resultiert ein Reliefbild, mit der letzten abgebildeten Stufe 3, 4 und 6.

**Patentansprüche**

**1.** Verbindungen der Formel I

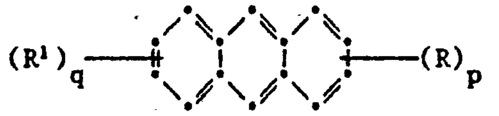

(I),

worin p 1 oder 2 ist, q 0 oder 1 darstellt, mit der Massgabe, dass die Summe p+q 1 oder 2 ist, jeder Substituent R unabhängig voneinander einen Rest der Formel II darstellt

$-A-(Q)_k$ (II),

worin Q einen Rest $-COZR^2$ darstellt, Z -O- oder $-NR^3-$ ist, und $R^2$ Wasserstoff, geradkettiges oder verzweigtes $C_1$-$C_{18}$Alkyl, geradkettiges oder verzweigtes $C_3$-$C_{18}$Alkenyl, $C_3$-$C_{12}$Cycloalkyl oder $C_7$-$C_9$ Aralkyl bedeutet, und $R^3$, wenn Z $-NR^3-$ ist, Wasserstoff oder geradkettiges oder verzweigtes $C_1$-

$C_{18}$Alkyl ist, oder $R^2$ und $R^3$ zusammen mit dem gemeinsamen Stickstoffatom einen fünf- oder sechsgliedrigen heterocyclischen Ring bilden; oder Q ein Rest -OX ist, worin X eine der Bedeutungen von $R^3$ annimmt oder -$COR^4$ ist, und $R^4$ Wasserstoff, geradkettiges oder verzweigtes $C_1$-$C_{18}$Alkyl, geradkettiges oder verzweigtes $C_3$-$C_{18}$Alkenyl oder $C_3$-$C_{12}$Cycloalkyl bedeutet, oder Q ein Rest -CN ist, worin k 1 oder 2 ist, A eine Gruppe der Formel III ist

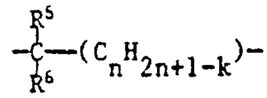

(III),

worin n eine ganze Zahl von 1 bis 17 bedeutet, k die oben definierte Bedeutung besitzt, $R^5$ und $R^6$ gleich oder verschieden sind und geradkettiges oder verzweigtes $C_1$-$C_5$Alkyl bedeuten, und, im Falle von Q = -$COOR^2$, $R^5$ oder $R^6$ gegebenenfalls eine -$COOR^2$ Gruppe tragen können, worin der Rest $R^2$ gleich oder unterschiedlich zum Rest $R^2$ der Gruppe Q ist, und wobei, im Falle der Substitution von $R^5$ oder $R^6$ mit -COOH, die Verbindung der Formel I nicht sehr als drei -COOH Gruppen trägt, oder worin $R^5$ oder $R^6$ so mit dem Rest $C_nH_{2n+1-k}$ verbunden sind, dass ein $C_5$-$C_{12}$Cycloalkylenring entsteht, der den Rest -$(COOR^2)_k$ trägt, worin die Reste $R^2$ und der Index k die oben angegebene Bedeutung besitzen, und der Substituent $R^1$ geradkettiges oder verzweigtes $C_1$-$C_8$Alkyl, $C_7$-$C_9$Aralkyl, das gegebenenfalls ein oder zwei Halogenatome als Substituenten trägt, oder eine ($C_1$-$C_4$)-Alkyl-($C_5$-$C_{12}$)-cycloalkylgruppe bedeutet; oder worin $R^1$ ein Rest der Formel II, wie oben definiert, ist, wobei im Falle $R^1$ = Rest der Formel II die Substituenten R und $R^1$ gleiche oder unterschiedliche Bedeutung besitzen können; sowie Salze der Verbindungen der Formel mit organischen oder anorganischen Basen.

2. Verbindungen der Formel I gemäss Anspruch 1, worin die Summe p + q 2 ist.

3. Verbindungen der Formel I gemäss Anspruch 2, worin R und $R^1$ jeweils einen Rest der Formel II darstellen.

4. Verbindungen der Formel I gemäss Anspruch 3, worin die Reste der Formel II identisch sind.

5. Verbindungen der Formel I gemäss Anspruch 4, worin die Reste R und $R^1$ sich in 2,6- oder 2,7-Position des Anthracengerüstes befinden.

6. Verbindungen der Formel IV gemäss Anspruch 1

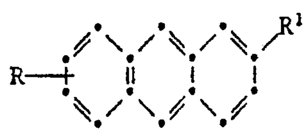

(IV),

worin R einen Rest der Formel II darstellt und $R^1$ ebenfalls einen Rest der Formel II darstellt oder $R^1$ ein geradkettiger oder verzweigtes $C_1$-$C_8$Alkylrest ist; sowie die Salze dieser Verbindungen.

7. Verbindungen der Formel I gemäss Anspruch 1, worin p 1 ist und q 0 bedeutet.

8. Verbindungen der Formel I gemäss Anspruch 1, worin k 1 ist.

9. Verbindungen der Formel I gemäss Anspruch 1, worin Q ein Rest -$COOR^2$ ist.

## Claims

1. A compound of formula I

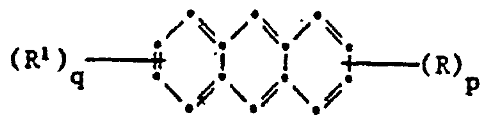

(I)

in which p is 1 or 2, q is 0 or 1, with the proviso that the sum of p+q is 1 or 2, each substituent R is independently a radical of formula II

$-A-(Q)_k$ (II) ,

in which Q is a radical $-COZR^2$ in which Z is -O- or $-NR^3-$, and $R^2$ is hydrogen, straight-chain or branched $C_1$-$C_{18}$alkyl, straight-chain or branched $C_3$-$C_{18}$alkenyl, $C_3$-$C_{12}$cycloalkyl or $C_7$-$C_9$aralkyl and when Z is $-NR^3-$, $R^3$ is hydrogen or straight-chain or branched $C_1$-$C_{18}$alkyl, or $R^2$ and $R^3$, together with the nitrogen atom to which they are both bonded, form a five- or six-membered heterocyclic ring; or Q is a radical -OX in which X has one of the meanings of $R^3$ or is $-COR^4$, and $R^4$ is hydrogen, straight-chain or branched $C_1$-$C_{18}$alkyl, straight-chain or branched $C_3$-$C_{18}$alkenyl or $C_3$-$C_{12}$cycloalkyl, or Q is a radical -CN in which k is 1 or 2, A is a group of formula III

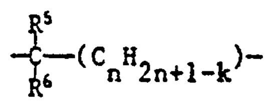

(III)

in which n is an integer from 1 to 17, k is as defined above, $R^5$ and $R^6$ are the same or different and each is straight-chain or branched $C_1$-$C_5$alkyl and, when Q is $-COOR^2$, $R^5$ or $R^6$ may optionally carry a $-COOR^2$ group, in which the radical $R^2$ is the same as or different to the radical $R^2$ of the group Q, and where, when $R^5$ or $R^6$ is substituted by -COOH, the compound of formula I does not carry more than three -COON groups, or in which $R^5$ or $R^6$ may be linked to the residue $C_nH_{2n+1-k}$ to form a $C_5$-$C_{12}$cycloalkylene ring which carries the radical $-(COOR^2)_k$, in which the radicals $R^2$ and the index k are as defined above, and the substituent $R^1$ is straight-chain or branched $C_1$-$C_8$alkyl, $C_7$-$C_9$aralkyl which is optionally substituted by one or two halogen atoms, or a $(C_1$-$C_4)$alkyl-$(C_5$-$C_{12})$cycloalkyl group; or in which $R^1$ is a radical of formula II as defined above, where, when $R^1$ is a radical of formula II, the substituents R and $R^1$ may be the same or different; and salts of the compound of formula I with organic or inorganic bases.

2. A compound of formula I according to claim 1, in which the sum of p+q is 2.

3. A compound of formula I according to claim 2, in which R and R' are each a radical of formula II.

4. A compound of formula I according to claim 3, in which the radicals of formula II are identical.

5. A compound of formula I according to claim 4, in which the radicals R and $R^1$ are in the 2,6- or 2,7-position of the anthracene structure.

6. A compound of formula IV according to claim 1

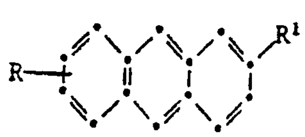

(IV)

in which R is a radical of formula II and $R^1$ is also a radical of formula II or $R^1$ is a straight-chain or branched $C_1$-$C_8$alkyl radical; and the salts of such a compound.

7. A compound of formula I according to claim 1, in which p is 1 and q is 0.

8. A compound of formula I according to claim 1, in which k is 1.

9. A compound of formula I according to claim 1, in which Q is a radical $-COOR^2$.

**Revendications**

1. Les composés de formule I ci-dessous :

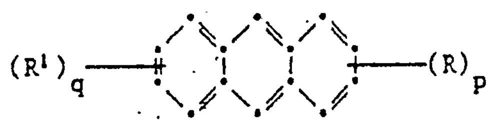

(I),

dans laquelle p est le nombre 1 ou 2 et q le nombre 0 ou 1, avec la condition que la somme p+q soit égale à 1 ou 2, chaque substituant R représente indépendamment de l'autre un radical de formule II

$-A-(Q)_k$ (II) ,

Q étant un radical $-COZR^2$, Z l'oxygène ou un groupe $-NR^3$ et $R^2$ l'hydrogène, un alkyle linéaire ou ramifie en $C_1$-$C_{18}$, un alcényle linéaire ou ramifié en $C_3$-$C_{18}$, un cycloalkyle en $C_3$-$C_{12}$ ou un aralkyle en $C_7$-$C_9$, et $R^3$, si Z est un groupe $-NR^3$-, représente l'hydrogène ou un alkyle linéaire ou ramifié en C1-C18, ou bien $R^2$ et $R^3$ forment ensemble et avec l'atome d'azote commun un hétérocycle pentagonal ou hexagonal ; ou bien Q est un radical -OX, X ayant l'une des significations indiquées pour $R^3$ ou étant un groupe $-COR^4$, $R^4$ étant l'hydrogène, un alkyle linéaire ou ramifié en $C_1$-$C_{18}$, un alcényle linéaire ou ramifié en $C_3$-$C_{18}$ ou un cycloalkyle en $C_3$-$C_{12}$, ou encore Q est un radical -CN, k est le nombre 1 ou 2 et A un groupe de formule III

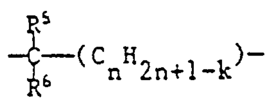

(III),

dans laquelle n est un entier de 1 à 17 et k le nombre 1 ou 2, et $R^5$ et $R^6$, qui peuvent être identiques ou différents l'un de l'autre, sont des alkyles linéaires ou ramifiés en $C_1$-$C_5$, et, si Q est un groupe $-COOR^2$, $R^5$ ou $R^6$ peut éventuellement porter aussi un groupe $-COOR^2$ dont $R^2$ est identique au ou différent du radical $R^2$ du groupe Q, et, s'il y a substitution de $R^5$ ou $R^6$ par le groupe -COOH le composé I n'a pas plus de 3 groupes -COOH, ou bien formule dans laquelle $R^5$ ou $R^6$ est lié au radical $C_nH_{2n+1-k}$ en formant un cycloalkylène en $C_5$-$C_{12}$ portant le radical - $(COOR^2)_k$ dont $R^2$ et l'indice k ont les significations qui ont été indiquées, et le substituant $R^1$ représente un alkyle linéaire ou ramifié en $C_1$-$C_8$, un aralkyle en $C_7$-$C_9$, pouvant éventuellement porter un ou deux atomes d'halogènes, ou un $(C_1$-$C_4)$alkyl-$(C_5$-$C_{12})$-cycloalkyle; ou bien $R^1$ est un radical de formule II ci-dessus définie, et dans ce dernier cas les substituants R et $R^1$ peuvent être identiques ou différents l'un de l'autre ; ainsi que les sels des composés de formule I formés avec des acides organiques ou minéraux.

2. Composés selon la revendication 1 dans lesquels la somme p+q=2.

3. Composés selon la revendication 2 dans lesquels R et $R^1$ sont des radicaux de formule II.

4. Composés selon la revendication 3 dans lesquels les radicaux de formule II sont identiques.

5. Composés selon la revendication 4 dans lesquels les radicaux R et $R^1$ sont aux positions 2,6 ou 2,7 du cycle de l'anthracène.

6. Composés selon la revendication 1, de formule IV :

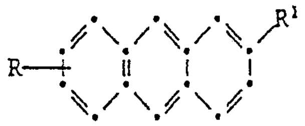

(IV),

dans laquelle R est un radical de formule II et $R^1$ également un radical de formule II ou bien un alkyle en $C_1$-$C_8$ à chaîne linéaire ou ramifiée, ainsi que les sels de ces composés.

**7.** Composés de formule I selon la revendication 1 dans lesquels p = 1 et q = 0.

**8.** Composés de formule I selon la revendication 1 dans lesquels k = 1.

**9.** Composés de formule I selon la revendication 1 dans lesquels Q est un radical -$COOR^2$.